Europäisches Patentamt

European Patent Office

Office européen des brevets

(19)

(11) Publication number: 0 248 088
A1

# EUROPEAN PATENT APPLICATION

published in accordance with Art. 158(3) EPC

(12)

(21) Application number: 86906937.7

(22) Date of filing: 19.11.86

Data of the international application taken as a basis:

(86) International application number:
PCT/JP86/00591

(87) International publication number:
WO87/03284 (04.06.87 87/12)

(51) Int. Cl.³: **C 07 K 15/04**
C 07 K 3/18, C 12 P 21/00
//A61K35/12, A61K37/02,
(C12P21/00, C12R1:91)

(30) Priority: 22.11.85 JP 261289/85
26.11.85 JP 263779/85

(43) Date of publication of application:
09.12.87 Bulletin 87/50

(84) Designated Contracting States:
AT BE CH DE FR GB IT LI NL SE

(71) Applicant: TEIJIN LIMITED
11 Minamihonmachi 1-chome Higashi-ku
Osaka-shi Osaka 541(JP)

(72) Inventor: FUKUMOTO, Yuji
609-3, Yokokawa-cho
Hachioji-shi Tokyo 193(JP)

(72) Inventor: OHTSU, Akira
13-35, Daimon 3-chome
Ohme-shi Tokyo 198(JP)

(72) Inventor: FUJII, Katsuhiko
2-18, Dai-machi 3-chome
Hachioji-shi Tokyo 193(JP)

(74) Representative: Votier, Sidney David
CARPMAELS & RANSFORD 43, Bloomsbury Square
London WC1A 2RA(GB)

(54) VASCULARIZATION FACTOR AND PROCESS FOR ITS PREPARATION.

(57) A vascularization factor which can be yielded by endo-
thelial cells of a blood vessel, which is absorbed by an
agarose gel having heparin immobilized thereon and is el-
uted by 0.7 M to 1.0 M sodium chloride, which is adsorbed by
a diethylaminoethyl anion exchanger and is eluted by 0.2 M
to 0.4 M sodium chloride, and which has a molecular weight
of about 5,000 to 10,000. This factor can be utilized in treating
ischemic diseases and wounds.

EP 0 248 088 A1

Croydon Printing Company Ltd.

SPECIFICATION

Title of the Invention

Angiogenic factor and Process for the Production Thereof

Technical Field

The present invention relates to a substance, which can be produced by the blood vessel endothelial cells and is capable of inducing angiogenesis, and a process for its production.

The object of this invention is to provide a peptide substance which is expected to be used as a remedy for ischemic diseases and as a therapeutic drug for wounds.

Background Art

The cells which compose the blood vessel are classified mainly into endothelial cells of tunica intima, smooth muscle cells of tunica media, and fibroblasts of tunica adventitia. The capillaries, however, do not have smooth muscle cells and fibriblasts, and build up their vessels only with endothelial cells basically. It may therefore be assumed that the growth of new capillary blood vessels is triggered off by the proliferation and migration of endothelial cells. The endothelial cells wholly cover the inner lumen of the vessel and are in contact with the blood flow. The maintenance of nonthrombogenic surfaces, adjustment of transport through the blood vessel, and also control of blood pressure may be counted among their functions. Furthermore, the rapid growth of capilaries (i.e. endothelial cells) is indispensable for the repair of an injury and the regeneration of a tissue. Speaking from the viewpoint of material production, the endothelial cells produce collagen, fibronectin, angiotensin II, and plasminogen activator to perform the very important functions mentioned in the above.

On the other hand, the endothelial cells which cover the blood vessel walls are in direct contact with the blood flow and accordingly often come off from the vessel walls because of damages caused by physical, chemical, and biological factors; however, very rapid repair and proliferation of endothelial cells site of their damage, thus making the blood vessel walls to keep on their homeostatic functions. However, once this repairing mechanism breaks down by some reasons, platelets adhere to the site of damage; which causes thrombus formation. It is also known that such damages increase the transpermeability through wall, followed by the interaction of some growth factors with smooth muscle cells to induce their proliferation causing the hypertrophy of tunica media and also promote the translocation of low density lipoprotein from blood plasma to the subendo-thelial tissue, thus allowing arteriosclerosis to develop. Such symptoms like this is generally regarded to lead sooner or later to more serious diseases such as myocardial infarction and cerebral infarction.

Now it is apparent that angiogenesis plays on important role in the repair and reconstruction of tissues at the sites where a thrombus is formed or where arteriosclerosis is found. Accordingly, the angiogenic factor which is capable of inducing new capillary growth is highly suggestive of becoming an effective drug for the aforementioned ischemic diseases by widely working upon the capillaries. Besides the abovementioned usage, the angiogenic factor foretells the possibility of its working as a therapeutic drug for wounds in view of the fact that the repair of wound after the major surgical operation, inclusive of organ transplantation, and the fixation of a transplated organ depend on how the new blood vessel can be formed at the site of operation.

It is known that a tumor requires to have nutritional blood vessels introduced thereinto to support its rapid growth and continues growing larger while releasing angiogenic factor, and accordingly the purification of an angiogenic factor has hitherto been performed entirely by use of tumor tissues or tumor cells as the starting material. With the object of obtaining a purified angiogenic factor from the normal tissues or cells to use such an angiogenic factor as a therapeutic drug for the aforementioned diseases, the present inventors paid exclusive attention to blood vessel endothelial cells which are most closely related to the neovascularization. It has been reported that, when the blood vessel endothelial cells are cultured successively under the appropriate conditions, the cells finally come to form a tube by themselves and this fact strongly suggests the possibility that the endothelial cells themselves produce an angiogenic factor. With regard to the angiogenic factor derived from the endothelial cells, the present inventors performed the endothelial cell migration test in vitro and the in vivo chick embryo chorioallantonic membrane assay and an accurate study to confirm the angiogenic activity in the culture supernatant, followed by a continued research on its purification, with the result of achieving the present invention.

Disclosure of the Invention

This invention relates to an angiogenic factor of molecular weight approximately 5,000 - 10,000, producible from blood vessel endothelial cells, having properties of being adsorbed to a heparin-agarose gel; being eluted with 0.7 - 1.0 M sodium chloride; and being adsorbed to diethylaminoethyl (DEAE) anion exchanger and eluted with 0.2 - 0.4 M sodium chloride.

This invention further relates to a process for the preparation of an angiogenic factor characterized in that the culture supernatant, which is obtained upon the serum-free culture of blood vessel endothelial cells, is applied to heparin-agarose gel, whose adsorbed active fractions are eluted with 0.7 - 1.0 M sodium chloride, and this active eluate is further applied to DEAE anion exchanger and the adsorbed active fractions are eluted with 0.2 - 0.4 M sodium chloride.

Brief Description of Drawings

Fig. 1 shows elution pattern obtained for the angiogenic factor of this invention by column chromatography an heparin-agarose gel and the migration activity of endothelial cells.

Fig. 2 shows the gel filteration pattern of the angiogenic factor and the migration activity of endothelial cells.

Fig. 3 shows the elution pattern of said angiogenesis factor obtained by DEAE-Sephacel chromatography.

Fig. 4 shows the biochemical properties of said angiogenesis factor.

Fig. 5 shows the elution pattern obtained for said angiogenesis factor by gel filtration.

Best Mode of Carrying Out the Invention

The angiogenic factor of this invention includes the ones produced by means of gene manipulation by use of facteria, yeasts, and animal cells so far as such products are substantially the same as those produced from the blood vessel endothelial cells. The angiogenic factors are generally produced from the culture supernatant resulting from the cultivation of blood vessel endothelial cells.

In the present invention, no limit is fixed as to the sourse of blood vessel endothelial cells and they can be collected from any part of human or animal blood vessels. Also no limit is placed upon the method and condition of cultivating the blood vessel endothelial cells and any method may be adopted; however, speaking from the easiness of purification, it is desirable first of all to use a method in which the endothelial cells are cultured in medium containing fetal bovine serum and thereafter the cultivation is conducted on serum-free medium until enough angiogenic factor is obtained.

The present inventors have also directed our intensive researches to the development of a method of producing the angiogenic factor in large quantity with much increased efficiency from the culture supernatant of blood vessel endothelial cells and have finally ascertained that the production of angiogenic factor is found much enhanced in the culture supernatant of adding a certain kind of angiogenic factor inducer to the culture system prepared for the blood vessel endothelial cells.

As the examples of such an inducer, natural prostaglandins such as prostaglandin $E_1$ and prostaglandin $A_1$ or their synthetic derivatives of nonnatural type; various kinds of peptides, which have a leukotactic activity, representing N-formyl-$\ell$-methionyl-$\ell$-leucyl-$\ell$-phenylalanine (FMLP) and N-formyl-$\ell$-methionyl-$\ell$-leucyl-$\ell$-phenylalanyl-$\ell$-phenylalanine; and activated vitamin D's such as 1$\alpha$,25-dihydroxychole-calciferol may be mentioned. Of these inducers mentioned above, the ones that are insoluble in water, are used in the form of an ethanol solution. The aforementioned inducers are used by being added to the medium in such a way as to make a concentration of $10^{-5}$ to $10^{-11}$ M, preferably $10^{-6}$ to $10^{-9}$ M. In case where the concentration of an inducer is less than $10^{-11}$ M, the effec-tiveness of this invention is no displayed enough and in case where it exceeds $10^{-5}$ M, the cytotoxicity is caused, both being contrary to the expected effect.

Since the angiogenic factor of this invention produced in the culture supernatant of the blood vessel endothelial cells has a nature of being adsorbed by heparin, the produced factor can be isolated and purified by use of heparin-agarose gel. Heparin is extracted from undenatured proteoglycan of swine intestinal mucosa and is sulfated glycosaminoglycan and binds to many kinds of coagulation factors, inclusive of antithrombin III, selectively. Several kinds of coagulation factors have, therefore, been isolated by employing their affinity to the immobilized heparin. Furthermore, heparin is interactive with many kinds of cationic substances because of its properties as polyanion. Such interactions accordingly establishes a basis for the purifying process by affinity chromatography conducted with the use of immobilized heparin. With regard to heparin-agarose gel proposed in this invention, the binding heparin to agarose-gel covalently can be conducted according to any of publicly known methods. As the commercial heparin-agarose gel, there are heparin-Sepharose CL-6B manufactured by Pharmacia and Affi-gel heparin manufactured by Bio-Rad Laboratories, but their sources are not limited to these nominated ones.

In the present invention, a buffer to be used for equilibrating with heparin-agarose gel in the adsorption of angiogenic factor should be a neutral buffer whose salt concentration is 0.5 M or less. Its pH value should preferably

be 6.5 to 7.5. It is preferable to keep the flow rate at 2 - 10 ml/hr cm$^{-2}$ in performing the adsorption of angiogenic factor to heparin-agarose gel and also to maintain the temperature at 0 - 10°C.

The elution of an angiogenic factor from heparin-agarose gel is performed with a linear gradient using, for instance, a neutral buffer containing 0.01 to 4.0 M, preferably 0.1 to 2.0 M sodium chloride. The said factor is eluted with 0.7 to 1.0 M sodium chloride. There is another method in which the stepwise elution is performed by using neutral buffers which respectively contain 0.7 M and 1.0 M sodium chloride. It is desirable to conduct the elution at a flow rate of 2 - 10 ml/hr cm$^{-2}$ and at a temperature ranging from 0 to 10°C.

DEAE anion exchanger is a weakly basic anion exchanger having tertiary ammonium base, diethylaminoethyl group, and $C_2H_4N^+(C_2H_5)_2H$. The fundamental theory of ion-exchange separation is based on a technique which utilizes the difference of binding ability to the ion exchanger. The ion exchange is a technique having a very high separating capability that can separate molecules of very weak electric charge difference and is now regarded to be one of the most reliable methods for purifying biological substances. As a support of the ion exchanger, high molecular polysaccharides such as cross-liked dextran-gel, cross-linked agarose-gel, and cellulose are used and a dissociative group is introduced into the matrix through covalent binding with a monosaccharide which forms the structural unit. Since DEAE anion

024808

exchanger has its surface charged positively, it is capable of adsorbing a substance whose net charge is negative and is, therefore, widely used for purifying such negatively charged substances. As the utilizable anion exchanger available on the market, there are DEAE-Sephacel, DEAE-Sephadex, and DEAE-Sepharose produced by Pharmacia, DEAE-Biogel A produced by Bio-Rad, and DE 52 produced by Whatman, but selection is not limited to these mentioned here.

The buffer which is used for the equilibration of DEAE anion exchanger at the time of adsorption of an angiogenic factor in the present invention is a neutral buffer whose salt concentration is less than 0.1 M. It is desirable to adjust its pH value at 7.0 to 8.0. It is also desirable to adjust the flow rate at the time of adsorption of angiogenic factor to DEAE anion exchanger at $2 - 10$ ml/hr $cm^{-2}$ and the temperature at 0 to 10°C.

The elution of angiogenic factor from DEAE anion exchanger is performed with a linear salt gradient using a neutral buffer which contains 0.02 to 1.5 M, preferably 0.1 to 0.7 M sodium chloride. The factor is eluted with 0.2 to 0.4 M sodium chloride. As another method of elution, a method in which the elution is conducted stepwise by using two neutral buffers, each containing 0.2 M and 0.4 M sodium chloride may also be available. The flow rate should preferably be $2 - 10$ ml/hr $cm^{-2}$ and the temperature should be 0 to 10°C.

It is estimated that the molecular weight of the angiogenic factor of this invention thus isolated and purified is approximately 5,000 - 10,000 when determined by a method of molecular sieve conducted with the use of insulin (molecular weight 6,000) and apolipoprotein A-II (molecular weight 8,500) as the standard substances.

The following Examples illustrate the invention.

Example 1

(1) Preparation of culture supernatant of bovine aortic endothelial cells (BAE)

Blood vessel endothelial cells were prepared from bovine arota according to known method (see Tissue Culture by Yoshi Mitsui, 1982, vol. 8; pp. 397 - 402), dispersed in Eagle's minimum essential medium (MEM) containing 10% fetal bovine serum (FBS), and performed the primary culture in a plastic Petri dish (10 cm∅) under the conditions of 5% $CO_2$ in air at 37°C. About one week later, the confluent cells were dispersed with 0.05% trypsin solution and passaged at a split ratio of 1 : 4. When the endothelial cells usually passaged between 2 and 10 reached to semi-confluency, the culture supernatant was removed and the cells were washed with serum-free Dulbecco's MEM medium (DMEM) containing no FBS and cultured again in serum-free DMEM. When 10 to 48 hours passed after the resumption of serum-free cultivation, the culture supernatant was collected and centrifuged. The obtained supernatant was dialyzed against 1 mM ammonium bicarbonate buffer (pH 7.6) and the dialyzate was lyophilized. The lyophilized product was dissolved in a 1/20 volume of a suitable buffer or a medium to be used as the starting material for an angiogenic factor.

(2) Angiogenic activity of BAE culture supernatant

The activity of the angionic factor in
the BAE culture supernatant prepared according to the aforementioned method was determined by the following two methods.

(i)　Migration test for blood vessel endothelial cells (in vitro)

Of the in vitro tests to study the neovascularization activity, the most important
one is the determination of the chemotaxis or
migration activity of the blood vessel endothelial cells which determines to direction of
capilary vascularization.

The migration activity of blood vessel
endothelial cells was measured according to
the method described by Albrecht-Buehler (Cell,
1977, vol. 11, pp. 395 - 404).

A cover glass with its surface coated
with colloidal gold was placed in a Petri dish
(3 cm$\emptyset$) which contained 500 $\mu \ell$ of 10% FBS-MEM,
to which 50 $\mu \ell$ of BAE culture supernatant and
500 $\mu \ell$ of $2 \times 10^3$ BAF suspension were added,
and the mixture was cultivated overnight at
37°C in 5% $CO_2$ air.

The migration activity was indicated on the
basis of the longest span of the migration track
of BAE observed under a microscope:  when the
longest span of the migration track is less than
the major axis of the cell, the index is 0, when
the longest span is 1 to 2 times the major axis,
the index is 1, when the longest span is 2 to 3
times the major axis, the index is 2, and when
the longest span is more than 3 times the major axis, the index is 3. As seen from Table 1, it has been made clear that, as compared with control, BAE culture supernatant has improved the migration activity of BAE.

(ii) Determination of neovascularization activity by chick embryo chorio-allantonic membrane assay (in vivo).

The determination of neovascularization activity in vivo was performed according to Folkman's method wherein neovascularization on the chorioallantonis of a fertilized hen's egg was utilized (Cancer Res., vol. 34, pp. 2109 - 2113). A small slit was opened on each fertilized hen's egg and a glass filter (GF/B made by Whatman) containing 20 µℓ of BAE culture supernatant was placed on the respective chorioallantonic membranes. After the eggs were incubated for 5 days in an incubator, the state of neovascularization was inspected. The frequency of strongly positive responses (+), quasipositive responses (±), and negative responses (-) were measured to determine the neovascularization activity. As shown in Table 2, the activity to promote the neovascularization was recognized in BAE culture supernatant.

Table 1

| Materials studied | Migration index |
|---|---|
| Control | 0.58 |
| Endothelial cells | 1.47 |
| Smooth muscle cells | 0.86 |

Table 2

| Materials studied | Neovascularization activity | | | Positive rate (%) |
|---|---|---|---|---|
| | + | ± | − | |
| Control | 0 | 2 | 7 | 0 |
| Endothelial cells | 5 | 2 | 3 | 50 |
| Smooth muscle cells | 0 | 0 | 3 | 0 |

Example 2

Purification of angiogenic factor with heparin-Sepharose

With the purpose of purifying the blood vessel angiogenic factor from BAE culture supernatant, the BAE culture supernatant prepared according to Example 1 was applied to heparin-Sepharose (Pharmacia) equilibrated with 0.01 M tris-hydrochloric acid buffer (pH 7.0) and 0.1 M sodium chloride and columns were eluted with linear gradient using 0.01 M tris-hydrochoric acid buffer (pH 7.0) containing 0.1 M to 2.3 M sodium chloride. The blood vessel endothelial cell's migration activities in each fraction are shown in Fig. 1. It was made apparent that the migration activity was found existing in the fractions eluted with 0.7 M – 1.0 M sodium chloride. The active fractions were dialyzed, concentrated, and the blood vessel angiogenic activity was determined by chick embryo chorioalantonic membrane test. As shown in Table 3, it has been made apparent that the fractions found with positive migration activity of endothelial cells also promote the neovascularization in vino assuredly.

Table 3

| Materials studied | Neovascularization activity | | | Positive rate (%) |
|---|---|---|---|---|
| | + | ± | − | |
| Control | 0 | 1 | 4 | 0 |
| Heparin-Sepharose adsorbed fraction | 4 | 1 | 3 | 50 |

Example 3

Purification of angiogenic factor by
molecular sieve column

The fractions of positive angiogenic activity partly purified with heparin-Sepharose were concentrated by lyophilization and then subjected to molecular sieve (flow rate 0.4 ml/min, at room temperature) by Superose 6 column (FPLC system manufactured by Pharmacia) equilibrated with 0.15 M ammonium formate buffer (pH 3.2). The migration activities of endothelial cells of each fraction were determined and the result is shown in Fig. 2. Insulin (molecular weight 6,000) and apolipoprotein A-II (molecular weight 8,500) were used as the molecular weight marker. As shown in Fig. 2, migration activity of endothelial cells was eluted almost at the same position as insulin with molecular weight of about 6,000 and the molecular weight of this angiogenic factor was found to be about 5,000 to 10,000. The active fractions were pooled and concentrated by

- 15 -

lyophilization and its angiogenic activity was examined _in vivo_ by the chick embryo chorio-allantonic membrane assay and its activity was confirmed as shown in Table 4.

Table 4

| Materials studied | Neovascularization activity | | | Positive rate (%) |
| | + | ± | - | |
|---|---|---|---|---|
| Control | 0 | 1 | 4 | 0 |
| Superose 6 active fraction | 3 | 0 | 3 | 50 |

Example 4

Purification of angiogenic factor by DEAE-Sephacel (made by Pharmacia)

The angiogenic factor which had partially been purified with heparin-Sepharose was dialyzed against 20 mM phosphate buffer (pH 7.4) containing 0.1 M sodium chloride and then applied to anion exchanger DEAE-Sephacel. After having been thoroughly washed, the factor was eluted with the linear gradient using a phosphate buffer (pH 7.4) containing 0.1 M to 0.6 M sodium chloride. As seen from Fig. 3 and Table 5, migration activity of endothelial cells was detected in the fractions eluted with 0.2 to 0.4 M sodium chloride and the neovascularization activity by the chick embryo chorioallantonic membrane assay was also found in the same fractions.

It has therefore been found that the angiogenic factor derived from the endothelial cells is adsorbed to anion exchanger DEAE-Sephacel
and is eluted with 0.2 to 0.4 M sodium chloride.

Table 5

| Materials studied | Migration index | Neovascularization activity |
|---|---|---|
| Control | 0.48 | - |
| Fraction A | 0.46 | - |
| Fraction B | 1.78 | + |

Example 5

Biochemical properties of angiogenic factor The angiogenic factor which had partially been
purified with heparin-Sepharose was subjected to
various treatments and their migration activities
were examined to clarify the biochemical properties.

(1) Acid treatment

Acetic acid of final concentration of 1 N and
1% formic acid were added to the angiogenic factor.
The mixture was left standing overnight at 4°C,
neutralized with 6 N NaOH, dialyzed against phosphate buffered saline (pH 7.2), their activities
were determined.

(2) Heat treatment

The factor was subjected to the heat treatment
at 100°C for 3 minutes, at 56°C for 30 minutes,
and at 37°C for 2 days respectively, directly
followed by the determination of their activities.

(3) Trypsin treatment

50 μg of trypsin (Sigma Chemical Co.) was
added to 500 μl of the factor and the mixture was incubated at 37°C for 2 hours. Thereafter, 100 μg of tripsin inhibitor (Sigma Chemical Co.) was added thereto and the activity was determined. A control was prepared by adding inactivated trypsin to the factor. More particularly, 50 μg of trypsin was pretreated with 100 μg of trypsin inhibitor at 37°C for 30 minutes beforehand and added to the factor. After the mixture was incubated at 37°C for 2 hours, the activity was determined.

(4) CM-Sephadex

The factor containing 0.15 M sodium chloride was dialyzed against 0.1 M phosphate buffer (ph 6.0) and applied to cation exchanger CM-Sephadex (Pharmacia) equilibrated with the same buffer and flow-through fractions and 1 M eluted fractions were collected. These fractions were concentrated by lyophilization, and after having been dissolved in a phosphate buffered (pH 7.2), the determination of activity was performed.

(5) Anti acidic FGF (fibroblast growth factor) antibody treatment

It was possible that the endothelial cell derived angiogenic factor was similler to acidic FGF, because both factors were eluted from heparin-Sepharose with almost the same concentration of sodium chloride. It was, therefore, studied if the activity of this angiogenic factor would be neutralized by the antibody against acidic FGF. More particularly, peptide having 15 terminal amino acid groups of acidic FGF was first synthesized and this peptide was bound to bovine serum albumin by use of water soluble carbodiimide and this was administered to rabbits for immunization to obtain anti-serum. Thus obtained anti-serum was passed through bovine serum albumin immobilized Sepharose to adsorb the antibody against bovine serum albumin. It was confirmed by the ELISA (enzyme-linked immunosorbent assay) that the antiserum thus obtained reacted specifically with peptide of acidic FGF only. The antiserum was subjected to 50% saturated ammonium sulfate precipitation treatment to give γ-globurin praction, which was then bound to affi-gel 10 (Bio-Rad Laboratories) to prepare an antibody immobilized column. The angiogenic factor was applied to this column and the activity of flow-through fraction was determined. A column prepared by binding normal rabbit γ-globulin to Affi-gel 10 was used as a control.

The result given in Fig. 4 shows that endothelial cell-derived angiogenic factor is peptide susceptible to trypsin. It has also been made apparent that this factor can not be adsorbed by CM-Sephadex (pH 6.0) and that it can not be neutralized with anti-acidic FGF antibody. The aforementioned result strongly suggests that this factor is a substance quite different from the known factors.

Example 6

    Purification of angiogenic factor by gel
    filtration under neutral conditions
The angiogenic factor, which had been partially purified with DEAE-Sephacel, was subjected to molecular sieve under the neutral conditions by Superose 12 column (FPLC made by Pharmacia) equilibrated with 50 mM phosphate buffer (pH 7.0) containing 1.0 M sodium chloride. The migration activities of endothelial cells of each fraction were measured giving the result as shown in Fig. 5, from which it was made apparent that the activity was found existing in the high molecular area of molecular weight 300,000 to 700,000. It is suggestive of the possibility that this factor forms a complex with the binding protein under the neutral conditions, judging from the fact that the activity is observed in the low molecular weight area of 5,000 to 10,000 under the acid conditions of pH 3.5.

Example 7

Acceleration of granulation tissue
formation with BAE culture supernatant
Firstly, 20 times concentrate of BAE culture supernatant was mixed with the equivalent volume of a slow-releasing polymer solution (10% hydroxypropylcellulose). Then a cotton pellet for dental use (White Cross Co., Ltd. No.3) was soaked with 200 µl of thus prepared mixed solution and air-dried. The air-dried cotton pellet was implanted subcutaneously at the shoulder blade of a 7-week Wister female rat. One week later, the cotton pellet was taken out and the weight of the granulation tissue formed around the pellet was measured. Another pellet soaked with MEM culture medium instead of BAE culture supernatant was used as a control and had its weight measured. As shown in Table 6, a significant increase of granulation tissue weight was observed with the cotton pellet soaked with BAE culture supernatant as compared with the control pellet.

Table 6

| Materials studied | Weight of granulation tissue    (mg) |
|---|---|
| Control | 279 |
| BAE culture supernatant | 395 |

Example 8

Promotion of would healing with BAE culture supernatant

50 µg of a hydrophilic ointment and 10 µl of 100 time concentrate of BAE culture supernatant were mixed for making BAE ointment. A would was made by incision as follows. A 7-week old Wister female rat had its back depilated, its skin incised with a flat chisel to make an incision to a length (long axis) of 1.5 cm. The incision was sewed up at its center and 50 mg of the BAE ointment was applied around the incision. The stiches was taken out after three days. Square shaped strips of skin divided into two parts at the center were cut from adheded incision, 3 days or 6 days after the forming of incision. The degree of wound healing was expressed by the tensile strength at the time of breaking adheded incision when the strip of skin was stretched. The degree of wound healing by use of the BAE ointment is shown in Table 7. It is apparent that the BAE ointment promoted the wound healing as compared with the control.

The results shown in Examples 7 and 8 indicate that the endothelial cell culture supernatant contains a factor which promotes the would healing and also supports the possibility of developing a wound healing drug by using this angiogenic factor.

Table 7

| Materials studied | Tensile strength (g) | |
|---|---|---|
| | 3 days | 6 days |
| Control (Hydrophilic ointment) | 149.1 | 378.7 |
| BAE ointment | 181.7 | 501.9 |

Example 9

Induction of angiogenic factor by various inducing agents

In the same way as adopted in Example 1, when the cultivation was resumed after the addition of serum-free DMEM, BAE was cultured in serum-free DMEM containing several kinds of angiogenic factor inducing agents respectively. The inducing activities of the angiogenic factors were determined by BAE migration test. As seen from Table 8, it has been made apparent that the stimulation of blood vessel endothelial cell with these inducing agents promotes the migration activity as compared with untreated culture supernatant. With regard to N-formyl-ℓ-methionyl-ℓ-leucyl-ℓphenylalanine, the angiogenic activity was determined by chick embryo chorioalantonic membrane test and as shown in Table 9, it has been made clear that the activity of angiogenic factor is also promoted in the FMLP treated culture supernatants.

Table 8

| Materials studied | Concentration (M) | Migration index |
|---|---|---|
| Control | – | 0.44 |
| BAE culture supernatant | – | 0.96 |
| 1.<br>PGE 1 treated culture supernatant | $3 \times 10^{-6}$ | 2.16 |
| 2.<br>EMLP treated culture supernatant | $10^{-7}$ | 1.78 |
| 3.<br>$1\alpha,25$-DHCC treated culture supernatant | $10^{-6}$ | 1.82 |

1.  Prostaglandin $E_1$

2.  N-formyl-$\ell$-methionyl-$\ell$-leucyl-$\ell$-phenylalanine

3.  $1\alpha,25$-dihydroxycholecalciferol

Table 9

| Material studied | Angiogenic activity | | | Positive ratio (%) |
|---|---|---|---|---|
|  | + | ± | – |  |
| Control | 0 | 2 | 2 | 0 |
| FMLP only | 2 | 1 | 3 | 33 |
| Culture supernatant | 3 | 2 | 2 | 43 |
| FMLP +<br>culture supernatant | 2 | 1 | 3 | 33 |
| FMLP treated<br>culture supernatant | 5 | 0 | 1 | 83 |

## Industrial Applications

The angiogenic factor of the present invention has an activity of inducing neovascularization. The formation of new blood vessels plays an very important role in the repair and reconstruction of tissues at the sites where thrombus is formed or where arteriosclerosis is found. Accordingly, the angiogenic factor of this invention is suggestive of becoming an effective drug for ischemic diseases by working upon the capillaries widely. In addition to the aforementioned usage, the angiogenic factor of this invention offers the possibility of its working as a therapeutic drug for wounds in view of the fact that the restoration of wound after the major surgical operation, inclusive of organ transplantation, and the fixation of a transplanted organ depend on how the new blood vessel can be formed at the site of operation.

Claims

1. An angiogenic factor of molecular weight approximately 5,000 - 10,000, producible from blood vessel endothelial cells, having properties of being adsorbed to a heparin-agarose gel; being eluted with 0.7 - 1.0 M sodium chloride; and being adsorbed to diethylaminoethyl anion exchanger and eluted with 0.2 - 0.4 M sodium chloride.

2. The angiogenic factor according to Claim 1 having properties of a peptide substance susceptible to trypsin.

3. A process for the preparation of an angiogenic factor characterized in that the culture supernatant, which is obtained upon the serum-free cultivation of blood vessel endothelial cells, is applied to heparin-agarose gel, whose adsorbed fractions are eluted with 0.7 - 1.0 M sodium chloride.

4. A process for the preparation of an angiogenic factor characterized in that the culture supernatant, which is obtained upon the serum-free cultivation of blood vessel endothelial cells, is applied to heparin-agarose gel, whose adsorbed fractions are eluted with 0.7 - 1.0 M sodium chloride and said eluate is further applied to the diethylaminoethyl anion exchanger and the adsorbed fractions are eluted with 0.2 - 0.4 M sodium chloride.

5. The process for the preparation of an angiogenic factor according to Claim 3 or Claim 4, characterized by the addition of $10^{-5} - 10^{-11}$ M inducing agent, which is capable of inducing the production of angiogenic factor, to the serum-free culture medium.

6. The process for the preparation of an angiogenic factor according to Claim 5, wherein said inducing agent is prostaglandin or its derivative.

7. The process for the preparation of an angiogenic factor according to Claim 5, wherein said inducing agent are peptides derived from bacteria, which are leukocyte chemoattractants.

8. The process for the preparation of an angiogenic factor according to Claim 5, wherein said inducing agent is an activated vitamin D.

# INTERNATIONAL SEARCH REPORT

International Application No. PCT/JP86/00591

## I. CLASSIFICATION OF SUBJECT MATTER (if several classification symbols apply, indicate all) [3]

According to International Patent Classification (IPC) or to both National Classification and IPC

Int.Cl[4] C07K15/04, 3/18, C12P21/00 //A61K35/12, 37/02 (C12P21/00, C12R1:91)

## II. FIELDS SEARCHED

### Minimum Documentation Searched [4]

| Classification System | Classification Symbols |
|---|---|
| IPC | C07K15/04, 3/18, C12P21/00, A61K35/12, 37/02 |

### Documentation Searched other than Minimum Documentation to the Extent that such Documents are Included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| A | JP, A, 57-206621 (Max-Plank-Gesellschaft zur Forderung der Wissenschaften e.V.) 18 December, 1982 (18. 12. 82) & US, A, 4465669 | 1-8 |
| A | JP, A, 60-11424 (Merck & Co., Inc.) 21 January 1985 (21. 01. 85) & US, A, 4444760 | 1-8 |
| A | Chemical Abstracts, Vol. 101, No. 3, 16 July 1984 (16. 07. 84) (Columbus, Ohio, U.S.A.) 18143n | 1-8 |

* Special categories of cited documents: [15]

"A" document defining the general state of the art which is not considered to be of particular relevance

"E" earlier document but published on or after the international filing date

"L" document which may throw doubts on priority claim(s) or which is cited· to establish the publication date of another citation or other special reason (as specified)

"O" document referring to an oral disclosure. use. exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents. such combination being obvious to a person skilled in the art

"&" document member of the same patent family

## IV. CERTIFICATION

| Date of the Actual Completion of the International Search [2] | Date of Mailing of this International Search Report [2] |
|---|---|
| February 4, 1987 (04. 02. 87) | February 23, 1987 (23. 02. 87) |

| International Searching Authority [1] | Signature of Authorized Officer [20] |
|---|---|
| Japanese Patent Office | |

Form PCT/ISA/210 (second sheet) (October 1981)